Europäisches Patentamt

European Patent Office

Office européen des brevets

(11) Veröffentlichungsnummer: **0 596 321 A2**

## (12) EUROPÄISCHE PATENTANMELDUNG

(21) Anmeldenummer: 93116944.5

(51) Int. Cl.5: **C07H 1/06**

(22) Anmeldetag: **20.10.93**

Ein Antrag gemäss Regel 88 EPÜ auf Hinzufügung von Tabellen liegt vor. Über diesen Antrag wird im Laufe des Verfahrens von der Prüfungsabteilung eine Entscheidung getroffen werden (Richtlinien für die Prüfung im EPA, A-V, 2.2).

(30) Priorität: **03.11.92 DE 4237105**

(43) Veröffentlichungstag der Anmeldung:
**11.05.94 Patentblatt 94/19**

(84) Benannte Vertragsstaaten:
**AT BE CH DE DK ES FR GB GR IE IT LI LU NL PT SE**

(71) Anmelder: **BEHRINGWERKE Aktiengesellschaft**
**Postfach 1140**
**D-35001 Marburg(DE)**

(72) Erfinder: **Hermentin, Peter**
**Salzköppel 9**
**D-35041 Marburg(DE)**
Erfinder: **Dönges, Reiner**
**Kreutzacker 6**
**D-35041 Marburg(DE)**
Erfinder: **Brazel, Dieter**
**Salzköppel 24**
**D-35041 Marburg(DE)**

(54) **Verfahren zur eindimensionalen Auftrennung und Identifizierung von Glycanen, sowie die Verwendung dieses Verfahrens zur Erstellung von Datenbanken und zur Strukturermittlung von Glycanen sowie zur relativen Ermittlung der Stokes'schen Radien von Glycanen.**

(57) Die Erfindung betrifft ein Verfahren zur genauen und reproduzierbaren eindimensionalen Auftrennung von Glycanen mittels der Kapillarelektrophorese, sowie die Verwendung dieses Verfahrens zur Erstellung von Datenbanken und zur Strukturermittlung von Glycanen, sowie zur relativen Ermittlung der Stokes'schen Radien von Glycanen.

EP 0 596 321 A2

Rank Xerox (UK) Business Services
(3.10/3.09/3.3.4)

Die Erfindung betrifft ein Verfahren zur genauen und reproduzierbaren eindimensionalen Auftrennung von Glycanen mittels der Kapillarelektrophorese, sowie die Verwendung dieses Verfahrens zur Erstellung von Datenbanken und zur Strukturermittlung von Glycanen sowie zur relativen Ermittlung der Stokes'schen Radien von Glycanen.

Vor allem auch im Zusammenhang mit gentechnisch hergestellten biologisch wirksamen Glycoproteinen wird die Strukturanalyse der Zuckeranteile, die bekanntlich wesentlich variabler sind als die Protein "backbones", immer bedeutungsvoller. Die Nachfrage nach einfachen, schnellen und vor allem gut reproduzierbaren Verfahren nimmt daher ständig zu.

Bekannte aufwendige Verfahren, die zudem ein hohes Maß an Expertise und instrumenteller Infrastruktur verlangen, sind z. B. die Methylierungsanalyse/GC-MS,FAB-MS und hochauflösende 'H-NMR-Spektroskopie.

In jüngerer Zeit wurde auch die Verwendung von chromatographischen Verfahren bekannt. Chromatographische Verfahren können allerdings nur dann zur Struktur-aufklärung eingesetzt werden, wenn entweder bei jedem Lauf alle bekannten (z. B. N-) Glycane als Standards mitlaufen gelassen werden, oder wenn für die bekannten Ver-bindungen Datenbanken erstellt werden können. Unerläßliche Voraussetzung für solche Datenbanken ist allerdings die hochpräzise und vor allem sehr gut reproduzierbare Messung der Retentions- bzw. Migrationszeiten. In Hermentin et al. (Anal. Biochem. (1992) 203, 281-289) wurde gezeigt, daß die Reproduzierbarkeit der Retentionszeit in der "high-pH anion-exchange chromatography with pulsed amperometric detection" (HPAE-PAD) für die Verwendung im Zusammenhang mit einer entsprechenden HPAE-PAD-Datenbank bei besser als 0.5% liegen muß. Die Trefferquote bei der Vermessung der nativen N-Glycane von rekombinantem Erythropoietin aus BHK-Zellen mittels HPAE-PAD lag beim Abgleich der gemessenen und korrigierten Retentionszeiten der einzelnen N-Glycane mit den Retentionszeiten der N-Glycane bekannter Struktur einer HPAE-PAD-Mapping-Datenbank bei kleiner als 0.7%.

Es hat sich jedoch gezeigt, daß sich die Reproduzierbarkeit beim Vermessen von N-Glycanen mittels HPAE-PAD als relativ schwierig erweist, da hierbei nicht nur apparativ bedingte Faktoren, sondern auch die Qualität der verwendeten Eluenten, die Wechselwirkung der zu trennenden Zucker mit den Anionenaustauscher-Gruppen, der zugrundeliegenden Polystyrol-Divinylbenzol-Matrix und den membranartig aufgezogenen Latex-Partikeln von erheblicher Bedeutung sind. Demgemäß spielen der jeweilige Zustand der Austauscher-Matrix, Regenerations- und Elutionsbedingungen, sowie Alterungs- oder Vergiftungsphänomene der Trennsäule für die zu erzielende Trennleistung eine wichtige Rolle.

Neuerdings findet die Kapillarelektrophorese (CE) zunehmend Anwendung zur Auftrennung und Charakterisierung von Mono-, Di- und Oligosacchariden und Glycanen.

Hierzu wird das Mono-, Di- oder Oligosaccharid oder Glycan am reduzierenden Ende mit einer Aminogruppen-haltigen UV- bzw. Fluoreszenz-aktiven Verbindung wie z. B. 2-Aminopyridin (Hase et al., J. Biochem. (1984) 95, 197-203) derivatisiert und fluorometrisch detektiert. Die erforderliche Reduktaminierung der Zucker, insbesondere der komplexen N-Glycane, gestaltet sich relativ aufwendig, erfordert zusätzliche Reinigungsschritte und führt in der Regel zu Ausbeuten von lediglich ca. 70% (Hase et al., J. Biochem. (1984) 95, 197-2039. Die Reduktaminierung Neuraminsäure-haltiger Glycane führt darüber hinaus zum partiellen Verlust terminal gebundener Neuraminsäure (Hase et al., J. Biochem. (1984) 95, 197-203), so daß diese Art der Derivatisierung für die Bestimmung nativer oder rekombinanter sialylierter Glycane nicht geeignet ist. Zudem führt selbst eine vorgeblich verbesserte Derivatisierungsmethode (Kondo et al., Agric. Biol. Chem. (1990) 54, 2169-2170) nicht zu quantitativen Ausbeuten.

Die Reduktaminierung mit z. B. 2-Aminopyridin beschränkt sich daher bislang auf die Derivatisierung neutraler Zucker und Glycane (siehe z. B. Honda et al., Anal. Biochem. (1990), 191, 228 - 234), oder die Aminopyridin-Derivate werden vor der Vermessung in der CE desialyliert (Suzuki et al., Anal. Biochem. (1992) 205, 227-236). In dem Falle, daß die Aminopyridin-Derivate sialylierter N-Glycane in der CE vermessen werden (Nashabeh und El Rassi, J. Chromatography (1991) 536, 31-43), wird der potentielle Verlust terminaler Neuraminsäure ignoriert.

Taverna et al. (Electrophoresis (1992) 13, 359-366) haben kürzlich gezeigt, daß sich N-Glycane aus Glycoproteinen in der CE in 50 mM Tricin/2.5 mM Putrescin, pH 8.2 (bei 20 °C) oder 200 mM Borat/1.25 mM Putrescin, pH 8.2 (bei 30 °C) gut auftrennen und bei 200 Nanometer detektieren lassen. Somit war ein Verfahren gefunden, welches die Vermessung sialylierter N-Glycane in der CE ohne Derivatisierung erlaubt. Allerdings mußte die Kapillare zur Erzielung der Trennungen mit 3-Mercaptopropyl-trimethoxysilan chemisch verändert worden.

Von Taverna et al. (ibid.) wurden keine Angaben zur relativen Standardabweichung der Migrationszeiten der vermessenen N-Glycane in der CE gemacht. Jedoch finden sich Angaben zur Reproduzierbarkeit der Retentionszeiten der detektierten Peaks (Glycoformen) des Glycopeptids "GP2" aus rekombinantem Gewebsplasminogen-Aktivator. Die elektrophoretische Mobilität wurde über Benzylalkohol als Marker für den elektroosmotischen Fluß kalkuliert. Die Standardabweichung der Retentionszeiten der Hauptpeaks in 6 aufeinanderfolgenden Läufen betrug bei Verwendung eines 150 mM Borat-Puffers, pH 9.0 - 9.2 weniger als 1.4% und bei Verwendung eines 100 mM Phosphat-Puffers, pH 6.6 zwischen 1.7 - 2.0%. In Gegenwart von 2.5 mM Putrescin betrug die relative Standardabweichung der Retentionszeiten der Hauptpeaks weniger als 2.3%, war also im Vergleich zu den Messungen ohne Putrescin leicht erhöht.

Suzuki et al. (Anal. Biochem. (1992) 205, 227-236) erzielten bei ihrer Vermessung von 2-Aminopyridin-Derivaten von asialo-N-Glycanen eine relative Standardabweichung der Migrationszeit von 0.86% in Phosphat-Puffer (100 mM Phosphat-Puffer pH 2.5, Gegenwart von 0.1% w/w Hydroxypropylcellulose) und von 1.03% in Borat-Puffer (200 mM Borat-Puffer pH 10.5). Die relative Standardabweichung der RT der N-Glycane konnte durch Korrektur über die Migrationszeit von Pyridyl-aminierter Glucose als internalem Standard auf 0.80% im Phosphat-Puffer und 0.50% im Borat-Puffer verbessert werden. Jeder Peak wurde durch Co-Elekrophorese mit der jeweils authentischen Probe bekannter Struktur identifiziert.

Es bestand daher die Aufgabe, die Kapillarelektophorese für N-Glycane so zu verbessern, daß auf die von Suzuki et al. verwendete Derivatisierung der N-Glycane verzichtet werden konnte (analog Taverna et al., ibid.), jedoch die relativ hohe Standardabweichung der Migrationszeit von Taverna et al. auf die von Suzuki et al. angegebene geringe Standardabweichung von 0.5 - 0.8% verbessert werden konnte.

Überraschender Weise wurde nun gefunden, daß sich sialylierte N-Glycane (ohne Derivatisierung) in einem Puffer, bestehend aus 80 mM Ammoniumsulfat, 20 mM Natriumphosphat und 2 mM 1,5-Diaminopentan oder 1,4-Diaminobutan, welcher mit Schwefelsäure oder Phosphorsäure auf pH 7.0 eingestellt wird, vorteilhaft bei einer konstanten Temperatur zwischen 25 und 35 °C, in der CE vermessen lassen, wobei sich eine Temperatur von 30 °C als optimal erwies.

Ferner wurde gefunden, daß sich sialylierte N-Glycane - unter Verzicht auf jegliche Derivatisierung - bei 190 nm bis hinab zu Konzentrationen im Femtomol-Bereich in der CE vermessen und detektieren lassen.

Weiter wurde gefunden, daß sich die Standardabweichung der Migrationszeiten (Retentionszeiten) in der CE auf unter 0.20% reduzieren läßt, wenn man die N-Glycane in Gegenwart zweier internaler Standards vermißt, die man so zu wählen hat, daß das zu vermessende N-Glycan zwischen den beiden internalen Standards liegt, und wenn man die RT des N-Glycans sowohl gegen den ersten als auch gegen den zweiten Standard korrigiert, mit der zuvor definierten Standard-Retentionszeit abgleicht, und aus den beiden korrigierten Werten den Mittelwert nimmt.

**Tabelle 1** zeigt 48 aufeinanderfolgende Trennungen in der CE des über Hydrazinolyse gewonnenen N-Glycan-Pools von humanem $\alpha_1$-saurem Glycoprotein (AGP). Die Mittelwerte der Retentionszeiten von 17 verschiedenen Peaks sind im Vergleich zu den jeweils korrigierten Werten und den jeweiligen Standardabweichungen und Variationskoeffizienten aufgeführt.

Man sieht, daß sich die Standardabweichung der unkorrigierten Peaks in den aufeinanderfolgenden Läufen zwischen 0.67% und 0.95% bewegt und somit vergleichbar zu den von Suzuki et al. (ibid.) für derivatisierte N-Glycane angegebenen Werten liegt, jedoch deutlich genauer ist als die von Taverna et al. (ibid.) für nicht derivatisierte N-Glycane angegeben Werte.

Die in Frage kommenden internalen Standards sind dadurch charakterisiert, daß sie einmal vor und einmal nach der zu vermessenen Probe in der CE erscheinen und unter den verwendeten Detektionsbedingungen detektiert werden.

Bei nicht derivatisierten Glycanen sind die zu verwendenden Standards dadurch charakterisiert, daß sie im Wellenlängenbereich zwischen 180 und 210 nm detektiert werden können, wobei eine Detektion im Bereich von etwa 190 nm bevorzugt ist.

Für die CE nicht derivatisierter sialylierter N-Glycane eignet sich als bevorzuger Standard mit früher Retentionszeit beispielsweise Mesityloxid. Jedoch können als Standards mit früher RT auch andere unpolare aromatische Verbindungen, die sich unter den CE-Bedingungen mit dem verwendeten Elektrophorese-Puffer vermischen, wie etwa Benzylalkohol, verwendet werden. Desweiteren kann selbst der sogenannte Puffer-Peak, welcher in der CE die Lauffront markiert bzw. ein Maß für den elektroosmotischen Fluß ist, als internaler Standard verwendet werden.

Als bevorzugter Standard mit später Retentionszeit eignet sich in der CE nicht derivatisierter sialylierter N-Glycane bevorzugterweise N-Acetyl-neuraminsäure (Neu5Ac). Jedoch können als Standards mit später RT auch andere zwischen 180 und 210 nm absorbierende und unter den Pufferbedingungen negativ geladene Verbindungen, wie beispielsweise N-Glycolylneuraminsäure oder Neuraminyl-Lactose oder im Puffer lösliche kurzkettige Carbonsäuren verwendet werden.

Weiter wurde gefunden, daß sich die Meßgenauigkeit in der CE entscheidend erhöhen läßt, wenn man die Korrektur der Meßwerte über die beiden internalen Standards auf möglichst genau definierte Standardwerte bezieht, die über Mittelwertbildung repetitiver Standardläufe ermittelt wurden, und das arithmetische Mittel der jeweils über die beiden internalen Standards korrigierten Meßwerte bildet. **Tabelle 1** belegt, daß die solcherart errechneten Mittelwerte der Retentionszeiten der 17 aufgelisteten Peaks der N-Glycane von AGP auf Variationskoeffizienten kleiner als 0.20% vermindert sind, während sich die Standardabweichung der unkorrigierten Peaks in den aufeinanderfolgenden Läufen zwischen 0.67% und 0.95% bewegt. Die Peak-Flächen und Peak-Höhen von Standard 2 (N-Acetylneuraminsäure) wurden in den 48 Läufen mit einer Standardabweichung von kleiner als 10% ermittelt. In einer separaten Versuchsserie mit 19 aufeinanderfolgenden Läufen konnten die Peak-Flächen und Peak-Höhen von Standard 2 jedoch mit einer Standardabweichung von kleiner als 2.5% detektiert werden (nicht gezeigt), was belegt, daß die CE auch verläßliche Konzentrationsbestimmungen von Glycanen erlaubt.

Weiter wurde gefunden, daß die über die beiden definierten Standards korrigierten und gemittelten Retentionszeiten so genau sind, daß sie den Aufbau einer validierten CE-Mapping-Datenbank sialylierter N-Glycane bekannter Struktur ermöglichen.

Ferner wurde gefunden, daß die auf diese Weise erstellte CE-Mapping-Datenbank so genau ist, daß sie die strukturelle Zuordnung der Peaks selbst bei Vermessung an verschiedenen, selbst Monate auseinanderliegenen Tagen und in verschieden langen Kapillaren erlaubt.

**Tabelle 2** belegt, daß die Wiederfindung der beispielhaft aufgelisteten N-Glycane der CE-Mapping-Datenbank nach Korrektur der Retentionszeiten in jedem Falle mit einem Variationskoeffizient kleiner als 0.20% erfolgt, während sich die unkorrigierten Retentionszeiten - trotz Vermessung in derselben Kapillare - im Mittel bisweilen um ca. 5% unterscheiden.

Obwohl die Korrektur der jeweils gemessenen Retentionszeiten der Proben über Mesityloxid und Neuraminsäure alleine die Variationskoeffizienten jeweils beträchtlich verbesserte, waren die dabei erhaltenen Weite für eine verläßliche und wiederholbar exakte Probenmessung noch deutlich zu hoch. Erst das arithmetische Mittel der beiden korrigierten Weite ergab für jede Probe einen nun dreifach korrigierten Weit, der jeweils eine Interassay-Variation von kleiner als 0.20% aufwies (**Tabelle 2**).

**Tabelle 3** zeigt, daß die Wiederfindung der beispielhaft aufgelisteten N-Glycane der CE-Mapping-Datenbank nach Korrektur der Retentionszeiten selbst dann mit einer mittleren Abweichung von kleiner als 0.20% exakt ist, wenn zwei Kapillaren verschiedener Länge, im gezeigten Beispiel etwa der halben bzw. doppelten Länge, verwendet werden.

Ferner wurde gefunden, daß die auf diese Weise erstellte CE-Mapping-Datenbank so genau ist, daß sie die strukturelle Zuordnung der N-Glycane natürlicher oder rekombinanter Glycoproteine ermöglicht, wobei die N-Glycane vom Glycoprotein auf enzymatischem Wege oder mittels Hydrazinolyse gewonnen sein können.

In diesem Zusammenhang wurde überraschender Weise gefunden, daß die über die beiden definierten Standards korrigierten und gemittelten Retentionszeiten der N-Glycane natürlicher oder rekombinanter Glycoproteine in der CE so genau sind, daß sie sich von den über die beiden definierten Standards korrigierten und gemittelten Retentionszeiten der N-Glycane der CE-Mapping-Datenbank stets weniger als 0.20%, meist weniger als 0,15% und oft selbst weniger als 0.10% unterscheiden.

**Tabelle 4** zeigt das Ergebnis der Strukturermittlung der auf enzymatischem Wege (mittels Glycopeptidase F bzw. PNGase F, Boehringer Mannheim, Tutzing, Deutschland) gewonnenen N-Glycane von rekombinantem Erythropoietin aus BHK-Zellen (Merckle GmbH, Ulm, Deutschland). Wie **Tabelle 4** zu entnehmen ist, konnten durch Abgleich mit der CE-Mapping-Datenbank 6 der insgesamt 7 in der CE detektierten Peaks strukturell zugeordnet werden - und zwar in 5 Fällen jeweils mit weniger als 0.25% Abweichung von den jeweiligen Weiten in der Datenbank. Nur in einem Falle betrug die Abweichung wegen der Überlagerung zweier Peaks zwischen 0.25 und 0.5%. Die Zuordnung der Peaks mittels CE war somit genauer und eindeutiger als die in der Literatur beschriebene Zuordnung der Peaks mit Hilfe einer HPAE-PAD-Mapping-Datenbank (Hermentin et al., Anal. Biochem. (1992) 203, 281-289).

**Tabelle 5** zeigt das Ergebnis der Strukturermittlung der über Hydrazinolyse mit dem GlycoPrep 1000™ (Oxford GlycoSystems, Abingdon, England) gewonnenen und über Sephadex G-25™ (Pharmacia, Uppsala, Schweden) entsalzten Glycane von Fetuin aus Rinderserum (Sigma, München, Deutschland). Wie **Tabelle 5** zu entnehmen ist, konnten durch Abgleich mit der CE-Mapping-Datenbank 6 der 9 in der CE detektierten Hauptpeaks strukturell zugeordnet werden - und zwar jeweils mit weniger als 0.25% Abweichung von den jeweiligen Werten in der Datenbank. Dabei konnten 3 dieser 6 Hauptpeaks sogar jeweils mit weniger als 0.10% Abweichung von den jeweiligen Werten in der Datenbank zugeordnet werden.

Somit wurde gefunden, daß sich die CE in hervorragender Weise zur Struktur-Zuordnung von N-Glycanen eignet und der Struktur-Zuordnung von N-Glycanen mittels HPAE-PAD sowohl bezüglich der Meßgenauigkeit und Reproduzierbarkeit als auch bezüglich der erforderlichen Menge an N-Glycan eindeutig überlegen ist. Es wurde nämlich weiterhin gefunden, daß zur Vermessung mittels CE etwa 4000 mal weniger N-Glycan-Material benötigt wird als zur Vermessung mittels HPAE-PAD unter den von Hermentin et al. (ibid.) beschriebenen Bedingungen.

4

Es liegt auf der Hand, daß sich die hier beschriebene hohe Genauigkeit und Reproduzierbarkeit der CE - bei der Verwendung zweier internaler Standards und der beschriebenen Korrektur - nicht nur auf die Vermessung nicht derivatisierter oder derivatisierter Glycanen beschränkt. Vielmehr läßt sich durch das hier beschriebene Verfahren die CE ganz allgemein, insbesondere jedoch bei der Vermessung von Peptiden und Glycopeptiden oder von Mono- oder Oligonucleotiden oder Nucleinsäuren oder deren Fragmente äußerst vorteilhaft anwenden und trägt somit erheblich zur vorteilhaften Anwendung der CE im Zusammenhang mit biochemischen und pharmakologischen Fragestellungen bei.

Im Falle der Vermessung derivatisierter Glycane eignen sich als internaler Standard 1 in der Regel dieselben Standards wie bei der Vermessung nicht derivatisierter Glycane oder auch das zur Derivatisierung der Glycane verwendete Reagenz selbst.

Als internaler Standard 2 eignet sich bei der Vermessung derivatisierter Glycane in der Regel ein analog zu den Glycanen derivatisiertes Monosaccharid, wie beispielsweise in analoger Weise derivatisierte Glucose oder Galactose.

Die Derivatisierung der Glycane, die mit der Einführung einer Ladung einhergeht, eignet sich bevorzugt zur Vermessung neutraler Glycane, wie z. B. von desialylierten Glycanen oder Glycanen vom sogenannten "high-mannose"-Typ.

Das beschriebene Verfahren eignet sich in analoger Weise auch zur Vermessung von Glycolipiden oder Gangliosiden oder von aus Glycolipiden oder Gangliosiden gewonnenen Glycanen, die ebenfalls in underivatisierter oder derivatisierter Form vermessen werden können.

Aufgrund der ausgezeichneten Reproduzierbarkeit und der damit verbundenen hohen Auflösung des erfindungsgemäßen Verfahrens konnte eine Methode zur Struktur-ermittlung auch unbekannter bzw. nicht in einer Datenbank enthaltenen Glycane ausgearbeitet werden.

Dabei kann die Strukturermittlung unbekannter bzw. nicht in einer Datenbank enthaltenen Glycane durch Exoglycosidase-Abbau (beispielsweise der derivatisierten Glycane) und Vermessung der dabei erhaltenen, in spezifischer Weise veränderten Glycane und den Abgleich der neu erhaltenen Retentionszeiten mit den Retentionszeiten einer Datenbank unterstützt werden.

Überraschenderweise zeigte sich dabei, daß die Migrationszeiten der Glycane bei gegebener Ladung mit zunehmendem Stokes'schen Radius kürzer werden.

Einige Regeln, die mit Hilfe der CE-Mapping-Datenbank sialylierter und nicht derivatisierter N-Glycane gefunden wurden, sind nachfolgend aufgeführt und in **Tabelle 6** schematisch zusammengefaßt, erheben jedoch keinen Anspruch auf Vollständigkeit:

Die Entfernung eines N-Acetylneuraminsäure-Restes vermindert die Retentionszeit (RT) eines N-Glycans um ~ 3 min für tetraantennäre (C4-4*) bzw. ~ 4 min für triantennäre (C3-3*) bzw. ~ 5 min für biantennäre (C2-2*) Strukturen.

Die Einführung eines oder zweier N-Acetyllactosamin-Repeats in C4-4*-Strukturen vermindert die RT um ca. 1.2 bzw. 2.2 min. Die Einführung eines N-Acetyllactosamin-Repeats in ein C4-3*-Isomer verkürzt die RT um ca. 0.9 min.

Analog verkürzt die Anknüpfung eines Galα1,3-Restes an OH-3 des β-glycosidisch verknüpften Galactose-Restes eines C4-3*-Isomers die RT um ca. 0.5 min. Und die Einführung zweier Galα1,3-Reste in ein C4-2*-Isomer verkürzt die RT um ca. 0.8 min.

In umgekehrter Weise führt die Entfernung eines β-Galactose-Restes zu einer Erhöhung der RT um ca. 0.5 min.

Die Abspaltung der proximal verknüften Fucose vermindert die RT um ca. 0.5 min.

Der Ersatz eines N-Acetylneuraminsäure-Restes durch einen N-Glycolylneuraminsäure-Rest führt zu keiner Änderung der RT.

Die Einführung einer Sulfatgruppe erhöht die RT um ca. 6-7 min.

Strukturelle Sialylisomere, welche Neu5Ac in α2,3- statt α2,6-Verknüfung zu β-Galactose enthalten, wandern pro Verknüpfungswechsel um ca. 0.3 min früher als die korrespondierenden α2,6-Isomere. Interessanter Weise ist dieses Verhalten umgekehrt wie bei der Vermessung mittels HPAE-PAD.

Die Neu5Acα2,3-verknüpften trisialo-triantennären fucosylierten Isomere (C3-335301.30.A4M3 und C3-335300.30.A6M6) sind in der CE um ca. 0.3 min getrennt (...A4M3 <...A6M6), während sich bei der Vermessung mittels HPAE-PAD keine Auftrennung der beiden Isomere erzielen läßt. Wiederum erweist sich somit die Vermessung in der CE der Vermessung mittels HPAE-PAD als überlegen.

Somit wurde für die Vermessung nicht derivatisierter sialylierter N-Glycane unter den bevorzugten und beispielhaft genannten Bedingungen folgende allgemeine Regeln erkannt:

Bei gegebener Ladung korrespondiert die Einführung eines Monosaccharid-Bausteins jeweils mit einer Verkürzung der Retentionszeit des betrachteten Glycans um ca. 0.5 - 0.6 min pro Monosaccharid-Baustein bzw. um ca. 1.0 - 1.2 min pro Disaccharid-Baustein.

5

So erlaubt beispielsweise die Beobachtung, daß der Wechsel von einer Neu5Ac$\alpha$2,3- zu einer Neu5Ac$\alpha$2,6-Verknüpfung bei einem N-Glycan zu einer Vergrößerung der Migrationszeit führt, die Schlußfolgerung, daß der Stokes'sche Radius bei einem N-Glycan mit $\alpha$2,3-verknüpfter Neu5Ac größer ist als bei dem korrespondierenden N-Glycan mit $\alpha$2,6-verknüpfter Neu5Ac.

Analog läßt sich überraschenderweise auch ableiten, daß der Stokes'sche Radius des triantennären Trisialo-Isomers C3-335301.30.A4M3 größer ist als der des korrespondierenden C3-335301.A6M6-Isomers, weil die Migrationszeit des ...A4M3-Isomers ca. 0.3 min kürzer ist als die des korrespondierenden ...A6M6-Isomers. Hierdurch eröffnen sich für die Kapillarelektrophorese im Verein mit einer validierten CE-Mapping-Datenbank völlig neue und überraschende forschungsmäßige Anwendungsmöglichkeiten, was den Weit der vorliegenden Erfindung in besonderer Weise belegt.

Darüber hinaus wurde die CE durch das beschriebene erfindungsgemäße Verfahren in Verbindung mit einer erfindungsgemäßen CE-Mapping-Datenbank als die schnellste, billigste, genaueste, verläßlichste, mit geringsten Mengen arbeitende und bis dato interessanteste und vielleicht zukunftsträchtigste Methode zur strukturellen Zuordnung sialylierter N-Glycane erkannt.

Das neue Verfahren zur Strukturermittlung von N-Glycanen mittels der CE unter Verwendung einer validierten CE-Mapping-Datenbank wird nachfolgend durch verschiedene Beispiele erläutert. Diese Beispiele schränken die Erfindung jedoch nicht ein.

**Legende zu den Tabellen und Abbildungen:**

Tabelle 1

Validierung der Genauigkeit und Reproduzierbarkeit CE-Läufe unter den in Beispiel 1 beschriebenen Pufferbedingungen durch 48-fache repetitive Injektion des mittels Hydrazinolyse aus $\alpha_1$-saurem Glycoprotein gewonnenen N-Glycan-Pools.

Die Mittelwerte der Retentionszeiten der 17 numerierten Peaks sind im Vergleich zu den jeweils korrigierten Werten und den jeweiligen Standardabweichungen und Variationskoeffizienten aufgeführt.
Standard 1: Puffer-Peak
Standard 2: N-Acetylneuraminsäure
RT, Retentionszeit; Stdabw, Standardabweichung; VK, Variationskoeffizient; Korr, nach Korrektur; Std, Standard

Tabelle 2

Wiederfindung der beispielhaft aufgelisteten N-Glycane der CE-Mapping-Datenbank nach Korrektur der Retentionszeiten - bei Vermessung an verschiedenen Tagen in derselben Kapillare

Tabelle 3

Wiederfindung der beispielhaft aufgelisteten N-Glycane der CE-Mapping-Datenbank nach Korrektur der Retentionszeiten - unter Verwendung zweier Kapillaren verschie-dener Länge (im gezeigten Beispiel etwa der halben bzw. doppelten Länge)

Tabelle 4

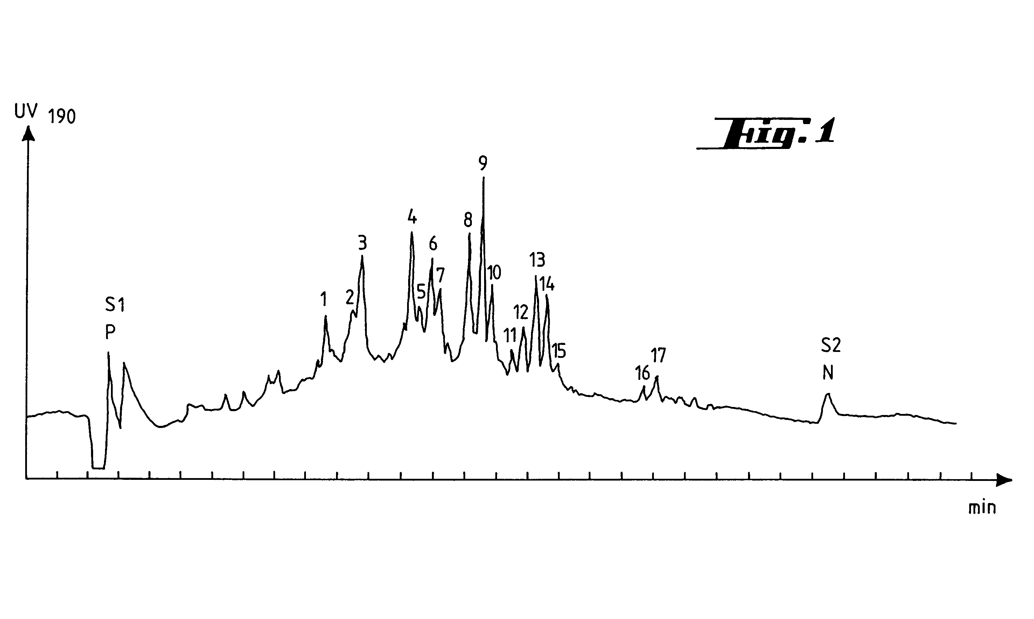

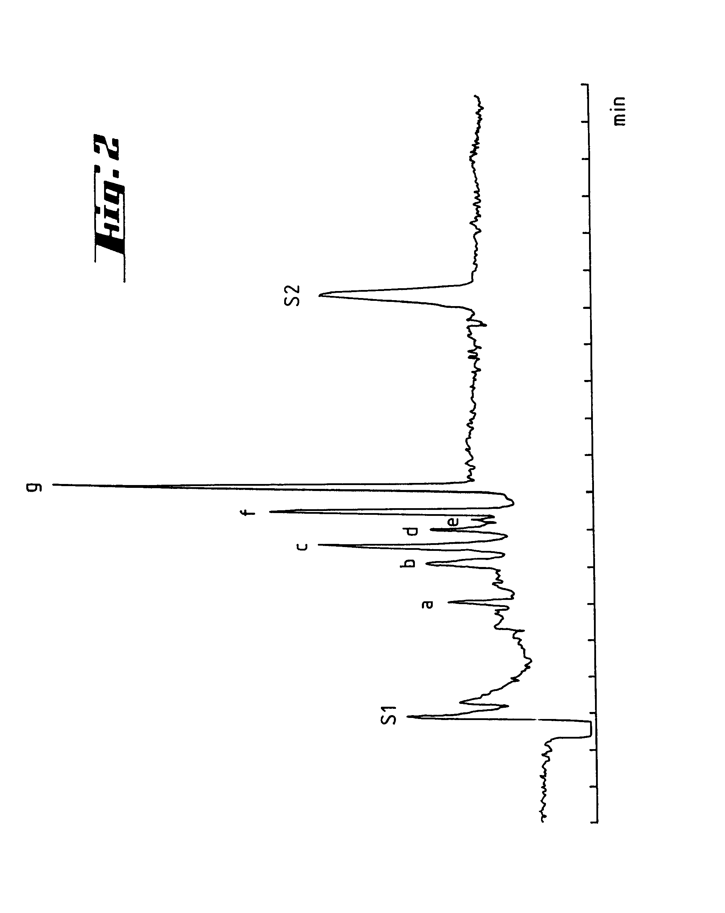

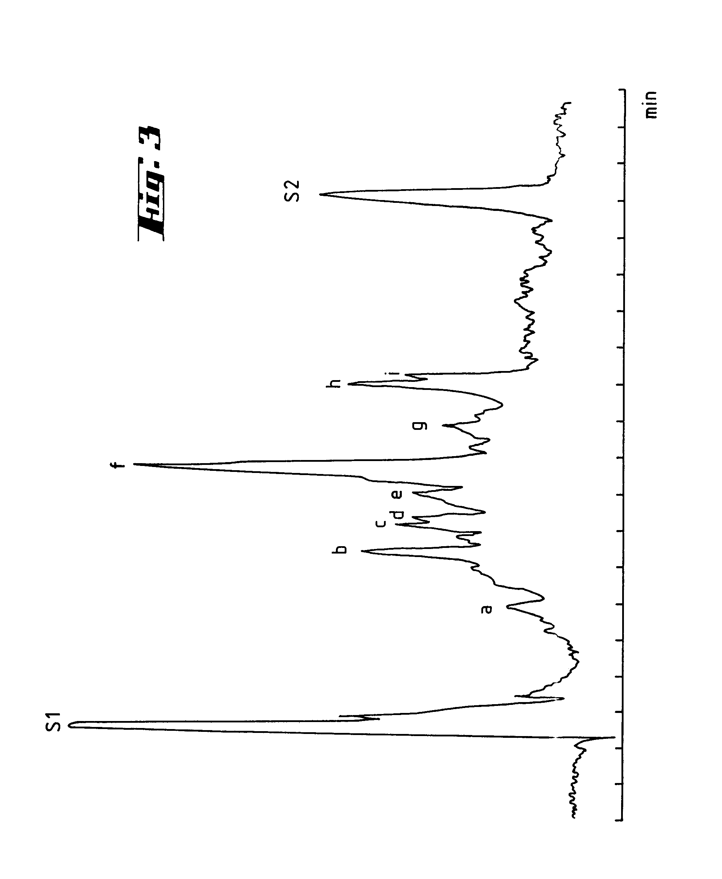

Strukturermittlung der auf enzymatischem Wege gewonnenen N-Glycane von rekombinantem Erythropoietin aus BHK-Zellen (CE-Lauf siehe Abbildung 2)

Tabelle 5

Strukturermittlung der mittels Hydrazinolyse gewonnenen Glycane von bovinem Fetuin (CE-Lauf siehe Abbildung 3)

Tabelle 6

Mit Hilfe der CE-Mapping-Datenbank gefundene Regeln für die strukturelle Zuordnung
von N-Glycanen unter Verwendung des in Beispiel 1 beschriebenen Puffers

Abbildung 1:

Beispielhafter CE-Läufe des mittels Hydrazinolyse aus $\alpha_1$-saurem Glycoprotein gewonnenen N-Glycan-Pools unter den in Beispiel 1 beschriebenen Pufferbedingungen.
Standard 1: Puffer-Peak
Standard 2: N-Acetylneuraminsäure
Die Ergebnisse der insgesamt 48 repetitiven Läufe sind in Tabelle 1 zusammengefaßt.

Abbildung 2:

CE-Lauf zur Strukturermittlung der auf enzymatischem Wege gewonnenen N-Glycane von rekombinantem Erythropoietin aus BHK-Zellen unter Verwendung des in Beispiel 1 beschriebenen Puffers (Auswertung siehe Tabelle 4).
Standard 1: Mesityloxid
Standard 2: N-Acetylneuraminsäure

Abbildung 3:

CE-Lauf zur Strukturermittlung der mittels Hydrazinolyse gewonnenen Glycane von bovinem Fetuin unter Verwendung des in Beispiel 1 beschriebenen Puffers (Auswertung siehe Tabelle 5).
Standard 1: Mesityloxid
Standard 2: N-Acetylneuraminsäure

**Beispiele:**

Die Beispiele beschränken sich auf sialylierte und nicht derivatisierte N-Glycane, jedoch ist die Erfindung nicht auf dieselben beschränkt.

Beispiel 1:

**Vermessung des mittels Hydrazinolyse aus $\alpha_1$-saurem Glycoprotein gewonnenen N-Glycan-Pools**

Die Isolierung des N-Glycan-Pools mittels Hydrazinolyse erfolgte in Auftragsarbeit durch die Firma Oxford GlycoSystems, Abingdon, England, in an sich bekannter Weise aus 50 mg $\alpha_1$-saurem Glycoprotein (Charge 281184, Behringwerke AG).
Die Vermessung des N-Glycan-Pools erfolgte mit einer Kapillarelektrophorese-Apparatur 270A-HT von Applied Biosystems unter Verwendung einer "fused-silica" Kapillare mit Innendurchmesser 50 $\mu$m, Außendurchmesser 365 $\mu$m und effektiver Länge 100 cm der Fa. Supelco, Bellefonte, PA, USA, im einem Puffer, bestehend aus 80 mM Ammoniumsulfat, 20 mM Natriumphosphat, 2.0 mM 1,5-Diaminopentan, eingestellt mit 1 N Schwefelsäure auf pH 7.0. Die Läufe erfolgten bei einer Temperatur von 30°C und einer Spannung von 20 kV bzw. einem resultierenden Stromfluß von 70 $\mu$A.
Die Läufe wurden bei 190 nm detektiert und mittels eines Perkin Elmer Interphase der Serie 900 unter Verwendung der Nelson PC-Integrator-Software, Version 5.1, aufgezeichnet.
Die neue Kapillare wurde vor den Läufen für 2 Stunden mit 1 M Natronlauge, 15 min mit Wasser und 30 min mit Elektrophorese-Puffer equilibriert.
Zwischen den einzelnen Läufen wurde die Kapillare jeweils für 5 min unter Verwendung eines 20-inch-Vakuums am kathodischen Kapillarenende mit dem Elektrophorese-Puffer gespült. Der Probenauftrag bzw. Auftrag der Standards erfolgte am anodischen Kapillarenende für 5 bzw. 2 sec durch Anlegung eines 5-inch-Vakuums.

Beispiel 2:

**Vermessung einzelner N-Glycane bekannter Struktur und Aufbau einer Mapping-Datenbank**

Ca. 60 N-Glycane wurden, wie in Hermentin et al. (Anal. Biochem. (1992), 203, 281-289) beschrieben, aus gereinigten Glycoproteinen isoliert und über Methylierungsanalyse, FAB-MS und 'H-NMR-Spektroskopie strukturell analysiert.

Weitere N-Glycane wurden, soweit erhältlich, von Oxford GlycoSystems, Abingdon, England, und von Dionex, Sunnyvale, USA, käuflich erworben.

Die Vermessung der Glycane erfolgte analog Beispiel 1, wobei jedoch anstelle einer Kapillare der Länge 100 cm bisweilen auch eine Kapillare der circa halben Länge verwendet wurde.

Der Aufbau der Datenbank erfolgte mittels des Kalkulationsprogramms Lotus 1-2-3.

Die verwendete Nomenklatur für N-Glycane ist wie in Hermentin et al. (Anal. Biochem. (1992), 203, 281-289) beschrieben.

Beispiel 3:

**Vermessung der N-Glycane von rekombinantem EPO aus BHK-Zellen**

Die Isolierung der N-Glycane erfolgte auf enzymatischem Wege unter Verwendung von Glycopeptidase F (PNGase F, Boehringer Mannheim, Tutzing, Deutschland) und Entsalzung mittels Sephadex G-25 (Pharmacia, Uppsala, Schweden), wie an anderer Stelle beschrieben (Hermentin et al., Anal. Biochem. (1992), 203, 281-289).

Die Vermessung in der CE erfolgte analog Beispiel 1.

Beispiel 4:

**Vermessung der Glycane von bovinem Fetuin**

Die Isolierung des Glycan-Pools mittels Hydrazinolyse erfolgte unter Verwendung des automatischen Hydrazinolyse- und Glycan-Isolierungsgeräts GlycoPrep$^{1000(TM)}$ der Firma Oxford GlycoSystems, Abingdon, England, mit ca. 2 mg bovinem Fetuin (Sigma, München, Deutschland) unter Verwendung der Originalchemikalien und Utensilien der Firma Oxford GlycoSystems und des apparativen Routineprogramms.

Die Vermessung in der CE erfolgte analog Beispiel 1.

**Patentansprüche**

1. Verfahren zur genauen und reproduzierbaren eindimensionalen Auftrennung und Identifizierung von Glycanen, das die folgenden Schritte einschließt:
   a) die Auftrennung erfolgt mit Hilfe der Kapillarelektrophorese,
   b) die Migration erfolgt dabei in einem Puffer ausgewählt aus der folgenden Gruppe von Puffern:
      i) Phosphatpuffer, 10-130 mMol/l, pH 2-8
      ii) Phosphat/Sulfatpuffer, 50-150 mMol/l, pH 4-10
      iii) Tricinpuffer, 20-100 mM/l, pH 6-10
      iv) Boratpuffer, 50-250 mM/l, pH 7-12;
   der Puffer enthält bevorzugt 0.5-2.5 mM/l eines oder mehrerer Diaminoalkane mit 2-6 Kohlenstoffatomen, bevorzugterweise 1,4-Diaminobutan und/oder 1,5-Diaminopentan,
      c) mit jeder Probe werden mindestens 2 Standardproben (interne Standards) chromatographiert
      d) die Detektion erfolgt bei derivatisierten Glycanen in den bekannten Wellenlängenbereichen, bei underivatisierten Glycanen in einem Wellenlängenbereich zwischen 180 und 210 nm, bevorzugt bei etwa 190 nm
      e) Auswertung der Retentionszeiten erfolgt nach Korrektur mit Hilfe der internen Standards (korrigierte Retentionszeiten), wobei die Standardabweichung der korrigierten Retentionszeiten (inter-assay-Variation in identischer Laufserie) besser als 0.25% ist.

2. Verfahren nach Anspruch 1, wobei die Standardabweichung der korrigierten Retentionszeiten besser als 0.20%, bevorzugt besser als 0.15%, besonders bevorzugt besser als 0.10% ist.

**3.** Verfahren nach Anspruch 1 oder 2, wobei die Glycane N-Glycane, bevorzugterweise sialylierte N-Glycane sind.

**4.** Verfahren nach mindestens einem der Ansprüche 1 bis 3, wobei 2 interne Standards verwendet werden, von denen einer vor und einer nach der Probe in der Elektrophorese erscheint.

**5.** Verfahren nach mindestens einem der Ansprüche 1 bis 4, wobei die Korrektur der gemessenen Retentionszeit(en) der Probe(n) über das arithmetische Mittel der über die beiden internen Standards korrigierten Retentionszeit(en) erfolgt.

**6.** Verfahren nach mindestens einem der Ansprüche 1 bis 5, wobei für die beiden internen Standards definierte Standardretentionszeiten dadurch festgelegt werden, daß die Retentionszeiten für die beiden Standards mehrfach ermittelt und dann jeweils das arithmetische Mittel daraus gebildet wird.

**7.** Verfahren nach mindestens einem der Ansprüche 1 bis 6, wobei die beiden Standards so gewählt sind, daß ein Standard zu Beginn und ein Standard am Ende der Elektrophorese erscheint.

**8.** Verwendung des Verfahrens nach mindestens einem der Ansprüche 1 bis 7 zur Erstellung einer Datenbank, die die Retentionszeiten bekannter Glycane enthält.

**9.** Datenbank, welche die mit einem Verfahren nach mindestens einem der Ansprüche 1 bis 7 ermittelten Retentionszeiten bekannter Glycane enthält.

**10.** Verwendung des Verfahrens nach mindestens einem der Ansprüche 1 bis 7 zur Identifizierung von Glycanen in einer zu untersuchenden Probe unter Verwendung einer nach Anspruch 8 erstellten Datenbank.

**11.** Verfahren zur Strukturermittlung von Glycanen, wobei die nach mindestens einem der Ansprüche 1 bis 7 bestimmten Retentionszeiten mit den Retentionszeiten bekannter Glycane in einer nach den Ansprüchen 8 oder 9 definierten Datenbank verglichen werden und daraus die Struktur des jeweils zu bestimmenden Glycans ermittelt wird.

**12.** Verfahren nach Anspruch 11, wobei die Glycane N-Glycane sind, bevorzugterweise sialylierte N-Glycane.

**13.** Verfahren nach Anspruch 12, wobei die Glycane N-Glycane vom komplexen Typ sind.

**14.** Verfahren zur relativen Ermittlung der Stokes'schen Radien von Glycanen, wobei die nach mindestens einem der Verfahren nach Anspruch 1 bis 7 ermittelte Retentionszeit mit einer aus der Retentionszeit mindestens eines bekannten Glycans gewonnenen Bezugswert verglichen und daraus der Stokes'sche Radius der untersuchten Glycane ermittelt wird.

**15.** Verfahren zur relativen Ermittlung der Stokes'schen Radien von Glycanen, wobei die nach mindestens einem der Verfahren nach Anspruch 1 bis 7 ermittelte Retentionszeit mit einer aus den Retentionszeiten bekannter Glycane gewonnenen Standardkurve verglichen und daraus der Stokes'sche Radius der untersuchten Glycane ermittelt wird.

EP 0 596 321 A2

Fig. 2

Fig. 3

EP 0 596 321 A2